# EUROPÄISCHE PATENTSCHRIFT

(11) **EP 1 227 751 B1**
(45) Veröffentlichungstag und Bekanntmachung des Hinweises auf die Patenterteilung: **29.12.2004**
(21) Anmeldenummer: 00964222.4
(22) Anmeldetag: 21.09.2000
(51) Int. Cl.: A61B 5/00

(54) **MOBILES ERGOSPIROMETRIESYSTEM**
MOBILE ERGOSPIROMETRY SYSTEM
SYSTEME MOBILE D'ERGOSPIROMETRIE

(30) Priorität: 09.11.1999 DE 19953866
(43) Veröffentlichungstag der Anmeldung: 07.08.2002
(73) Patentinhaber: Cortex Biophysik GmbH, 04229 Leipzig (DE)
(72) Erfinder: GEHRKE, Matthias, 60318 Frankfurt/Main (DE); HENKER, Ralf, 04207 Leipzig (DE); KRETSCHMER, Claus-Peter, 04347 Leipzig (DE); KRIESMER, Thomas, P., 56626 Andernach (DE)
(74) Vertreter: Kruspig, Volkmar, Dipl.-Ing.
(86) Internationale Anmeldenummer: PCT/EP2000/009259
(87) Internationale Veröffentlichungsnummer: WO 2001/034022

(56) Entgegenhaltungen:
- EP-A- 0 846 440
- WO-A-98/53732

## Beschreibung

Die Erfindung betrifft ein mobiles Ergospirometriesystem, umfassend eine am Probanden fixierbare, tragbare Meßeinheit mit Mundstück oder Maske zur Atemgasabnahme, Gasvolumen- oder Mengensensor sowie Sensoren zur Bestimmung der CO₂/O₂-Konzentration im Atemgas, einen Signalverarbeitungsprozessor und ein Telemetriemodul sowie weiterhin eine computergestützte Basisstation mit Telemetrieeinheit zum Aufbau einer drahtlosen Verbindung mit dem Telemetriemodul der tragbaren Meßeinheit, gemäß Oberbegriff des Patentanspruchs 1.

Mobile Ergospirometriegeräte z.B. für Belastungsuntersuchungen außerhalb des Labors sind seit einigen Jahren bekannt. Mit derartigen mobilen Systemen lassen sich Analysen direkt auf dem Sport- oder am Arbeitsplatz unter natürlichen Bedingungen und Belastungssituationen durchführen. Über Telemetrieeinheiten werden die Meßdaten in Echtzeit an einen Personal-Computer oder ein Notebook übertragen, wobei eine entsprechende Steuerung des Trainings- oder Übungsverlaufs nach Auswertung der Daten möglich ist. Durch derartige Geräte wurden neue Anwendungsgebiete in der Leistungsdiagnostik, in Arbeits-, Sport- und der Rehabilitationsmedizin erschlossen

Aus der PCT-WO 98/53732 ist ein tragbares Ergospirometriesystem mit einer Telemetrie-Datenübertragungseinheit bekannt. Mit der dort gezeigten Anordnung sollen individuelle Parameter hinsichtlich der Sauerstoffaufnahme und der Kohlendioxidproduktion erfaßbar, d.h. eine Atemanalyse in Verbindung mit der Bestimmung des Herzrhythmus unter natürlichen Umgebungsbedingungen möglich sein.
Gemäß der bekannten Lehre besitzt eine tragbare Einheit, die am Probanden befestigt ist, neben einer Atemmaske eine Gasanalyseeinrichtung zur Bestimmung der O₂- und CO₂-Werte des Meßgases. Zusätzlich ist ein Herzfrequenzmonitor vorgesehen, wobei die Meßwerte einem Mikroprozessor zugeführt werden, der Bestandteil der tragbaren Einheit ist. Die Meßwerte werden in einem internen Speicher abgelegt und über eine Telemetriestrecke zu einem Telemetrieempfänger übertragen, der wiederum mit einem Personal-Computersystem in Verbindung steht.

Der Volumenfluß des Atemgases wird nach PCT-WO 98/53732 mit einem speziellen Flowmeter bestimmt, welches rotierende Flügel besitzt, deren Bewegung über eine Infrarot-Lichtemitter-Diodenstrecke abgetastet wird.

Die Telemetrie-Datenübertragungsstrecke zwischen der tragbaren Einheit und der Basisstation nebst Personal-Computer dient der Übernahme von Meßdaten des Ergospirometers zur Anzeige der Daten, späteren Auswertung und Bearbeitung.

Eine aktive Einflußnahme auf den Versuchsablauf seitens eines leitenden Arztes, mit dem Ziel Diagnostikwerte zu verbessern, ist beim bekannten Stand der Technik ohne weiteres nicht möglich.

Darüber hinaus ist das bekannte tragbare System in seiner Anwendung aufwendig und umständlich, wobei die Einweisungszeit bei häufig wechselnden Probanden zu hoch ist.

Es ist daher Aufgabe der Erfindung, ein weiterentwickeltes mobiles Ergospirometriesystem, umfassend eine am Probanden fixierbare, tragbare Meßeinheit sowie eine computergestützte Basisstation anzugeben, wobei mit diesem System eine unmittelbare Online-Beeinflussung und Optimierung des Versuchsablaufs möglich sein soll und gleichzeitig die Akzeptanz derartiger Systeme sowohl beim Probanden als auch bei der für die Versuchsdurchführung verantwortlichen Person erhöht wird. Zusätzlich soll die Möglichkeit bestehen, erhaltene Meßwerte in der tragbaren Meßeinheit speicherplatzoptimal abzulegen, so daß auch beim temporären Ausfall einer Datenübertragungs-Telemetriestrecke ein Datenerhalt gegeben ist, wodurch wiederum die Anzahl der Versuche und damit die Belastung des Probanden minimiert werden kann.

Die Lösung der Aufgabe der Erfindung erfolgt durch ein mobiles Ergospirometriesystem gemäß den Merkmalen des Patentanspruchs 1, wobei die Unteransprüche mindestens zweckmäßige Ausgestaltungen und Weiterbildungen umfassen.

Demgemäß besteht der Grundgedanke der Erfindung darin, eine spezielle bidirektionale Telemetriestrecke mit einem Sprachmodul als Bedienhilfe anzugeben, wodurch Informationen oder Aufforderungen zur Bedienung der Meßeinheit und/oder dem Versuchsverlauf von der für den Versuch verantwortlich leitenden Person übermittel-, d.h. zum Probanden hin übertragbar sind. Auch ist eine akustische Ausgabe von gerätetechnischen Informationen, z.B. nach einem Selbsttest, z.B. "Atemgasschlauchdefekt" möglich.

Erfindungsgemäß ist der Signalprozessor der tragbaren Meßeinheit in der Lage, empfangene, codierte Befehle auf einen Sprachmodul zur Umsetzung zu leiten, wobei das Sprachmodul Audiosignale von Verhaltenshinweisen oder dergleichen Anweisungen, aber auch Meßdaten ausgibt. Diese Signale können dann beispielsweise mittels eines Ohrhörers oder eines anderen akustischen Wandlers dem Probanden zur Verfügung gestellt werden.

Die tragbare Meßeinheit ist so ausgeführt, daß diese sämtliche Funktionen eines Ergospirometriesystems einschließlich Berechnung und Speicherung von Meßwerten sowie zur Steuerung der Bedien-, Meß- und Kommunikationsfunktionen erfüllt.
In einer bevorzugten Ausführungsform der Erfindung weist die Telemetrieeinheit der Basisstation eine automatische Frequenzwähleinrichtung auf, um nach Scannen des Frequenzbands und/oder einer Testdaten-Übertragungsprozedur und Auswertung des Übertragungsergebnisses eine Telemetriefrequenz mit erwarteter optimaler Übertragungsqualität festzulegen. Diese Testübertragung kann mit Versuchsbeginn erfolgen, wobei auch eine wiederholte Überprüfung anhand einer z.B. Feldstärkenüberwachung der ankommenden Signale denkbar ist.

Neben dem in die Meßeinheit integrierten Herzfrequenzdetektor ist ein mehrkanaliges EKG-Modul vorhanden, wobei die Erfassung der ergospirometrischen und der kardiologischen Meßwerte unter Rückgriff auf den Signalprozessor synchronisiert erfolgt. Durch diese synchronisierte Meßdatenerfassung und Darstellung ist der unmittelbare zeitliche Zusammenhang der Werte erkennbar und es erhöht sich die diagnostische Relevanz und der Aussagegehalt der Daten.

Für die Zwischenspeicherung insbesondere mit Blick auf die Datensicherung ist in der Meßeinheit ein Speicher vorgesehen, wobei im Speicher Datensätze in einem wählbaren Format entweder atemzugsbezogen oder über einen vorgebbaren Zeitraum bzw. ein Zeitintervall abgelegt werden.

Der Signalprozessor ermöglicht eine Berechnung der Ergospirometriegrößen aus den vorliegenden Daten, wobei diese berechneten Daten im Speicher abgelegt und/oder dem Probanden dargestellt bzw. übermittelt werden.

Der Signalprozessor ist in der Lage, aufgrund eines externen Befehls oder automatisch unter Berücksichtigung vorliegender Meßverläufe oder Meßdaten unter Berücksichtigung des geplanten Versuchsverlaufs Verhaltenshinweise zu ermitteln und diese über das Sprachmodul an den Probanden auszugeben. Hierfür enthält das Sprachmodul einen digitalen Speicher mit dort abgelegten Worten, Wortgruppen oder sonstigen akustisch darstellbaren Informationen.

Mit Hilfe des Signalprozessors kann aus den im digitalen Speicher abgelegten Worten oder Wortgruppen eine jeweils aktuelle befehlsorientierte Wortverbindung ausgewählt, verknüpft und gesteuert ausgegeben werden.

Über zusätzliche Sensoren in der tragbaren Meßeinheit z.B. zur Bestimmung der Umgebungstemperatur und/oder der Atemgasfeuchte kann eine Kalibrierung und Korrektur der Atemgas-Sensorwerte durchgeführt werden.

Neben der Möglichkeit der telemetrischen Datenübertragung besitzt die tragbare Meßeinheit eine Standard-Schnittstelle zur bevorzugt drahtgebundenen Übertragung von Daten hin zur Basisstation oder zu einem mit einer entsprechenden Auswertesoftware versehenen Personal-Computer.
Die Basisstation weist eine Schnittstelle zur Herstellung einer Verbindung mit einer als Zusatzbaugruppe ausführbaren bidirektionalen Telemetrieeinheit auf, so daß z.B. die Basisstation in Form eines Personal-Computers am Arbeitsplatz der versuchsleitenden Person aufgestellt werden kann, wobei die Telemetrieeinheit davon abgesetzt an einer zum Aufbau der Hochfrequenz-Übertragungsstrecke optimalen Position befindlich ist.

Die tragbare Meßeinheit ist funktional so aufgeteilt, daß in zwei nahezu gleichschweren, gleichgroßen Gehäusen sämtliche Hardwarekomponenten untergebracht sind. Beide Gehäuse sind durch ein Kabel miteinander verbunden. Über ein Tragesystem wird die zweigeteilte Meßeinheit im Bereich der Schultern/Schlüsselbeine vom Probanden getragen, so daß dieser in seiner Bewegung nahezu uneingeschränkt ist und die Meßeinheit nicht als zusätzliche Belastung empfindet. Dadurch, daß der Befestigungspunkt weit am Oberkörper gewählt ist, können Zuleitungen zwischen dem Mundstück oder der Maske recht kurz ausgeführt werden und die Antennenabstrahlfunktion des Telemetriemoduls ist optimal.

Weiterhin bevorzugt sind Sekundärelemente zur Stromversorgung in einem der Gehäuse integriert. Demnach ist ein weiteres vom Probanden zu tragendes Element mit Akkumulatoren und entsprechenden Verbindungskabeln hin zur eigentlichen Meßeinheit nicht notwendig.

An mindestens einem der Gehäuse sind Bedien- und Anzeigemittel, z.B. in Form von Folientasten in Verbindung mit lichtemittierenden Dioden vorhanden, so daß der Proband in der Lage ist, mit der Meßeinheit audiovisuell zu kommunizieren und die Einheit zu bedienen.

Die Signalvorverarbeitung respektive die Berechnung der Ergospirometriegrößen in der tragbaren Meßeinheit selbst ist so angelegt, daß der Proband direkt von der Meßeinheit aus informiert werden kann. Auf diese Weise kann die tragbare Meßeinheit auch autark betrieben werden, wobei die Meßdaten im erwähnten Speicher abgelegt sind. Eine nachträgliche Auswertung durch Herunterladen der Daten ist dann ohne weiteres möglich.
Der Speicher zur Datensicherung in der tragbaren Meßeinheit ist so ausgelegt, daß die Speicherinhalte auch nach Abschalten der Einheit über einen definierten Zeitraum erhalten bleiben. Die tragbare Meßeinheit besitzt über den Signalverarbeitungsprozessor die Möglichkeit, bei erkannter unterbrochener Telemetrie-Datenübertragung gänzlich auf eine interne Speicherung umzuschalten, ohne daß eine Wiederholung der ergospirometrisch relevanten Übung notwendig ist. Die Meßdaten werden jedoch grundsätzlich in einem Zwischenspeicher gepuffert.

Bei einer Ausgestaltung der Erfindung kann die Meßeinheit weitere Schnittstellen zum Anschluß von zusätzlichen Geräten oder Sensoren aufweisen, mit deren Hilfe beispielsweise vitale Körperfunktionen über Ermittlung des Blutdrucks, der Hauttemperatur oder dergleichen bestimmt und im Versuchsablauf berücksichtigt werden können.

Die Sprachsteuerung der tragbaren Meßeinheit ist durch softwareseitige Intelligenz in die Lage versetzt, digital gespeicherte und/oder synthetisch erzeugte Worte oder Wortgruppen unmittelbar auszugeben, aber auch mit Bezug auf bestimmte Aufforderungen oder Befehle zu verknüpfen. Diese Befehle können vom Versuchs- bzw. Testleiter über das Telemetriemodul der Basisstation ausgelöst, jedoch aber auch automatisch von der Meßeinheit selbst unter Berücksichtigung aktueller Meßoder Steuerungswerte generiert werden.

Die Erfindung soll nachstehend anhand eines Ausführungsbeispiels sowie unter Zuhilfenahme von Figuren näher erläutert werden.

Hierbei zeigen:
- Fig. 1: typische Funktionseinheiten eines Ergospirometriesystems;
- Fig. 2: ein Blockschaltbild der tragbaren Meßeinheit;
- Fig. 3: die Basisstation mit Telemetriemodul und
- Fig. 4: eine Darstellung eines mobilen Ergospirometriesystems, wie es mit den beiden Gehäusekomponenten über ein Tragesystem am Körper eines Probanden befestigbar ist.

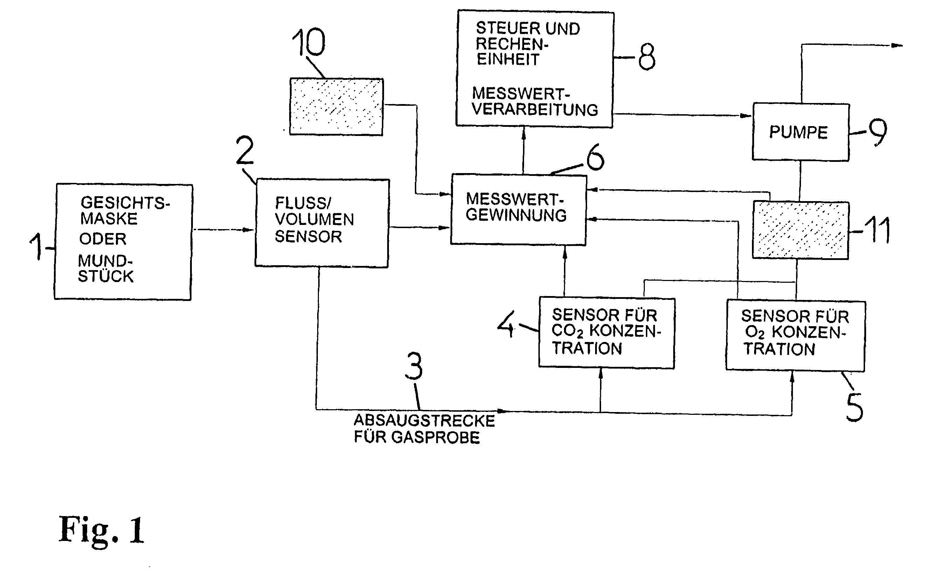

Ein Ergospirometriesystem, wie in der Fig. 1 dargestellt, geht von einer Gesichtsmaske oder einem Mundstück 1 aus, welches Atemgas über einen Fluß/Volumensensor 2 und eine Absaugstrecke 3 den Sensoren für die Bestimmung der CO₂- und O₂-Konzentration 4; 5 zuführt.
Prinzipiell sind mit einer entsprechenden Atemgasführung Meßwerte im Breath-by-Breath-Verfahren, aber auch durch Nutzung einer Gasmischkammer zu erhalten. Aus Gründen der angestrebten Optimierung einer ergospirometrischen Analyse mit entsprechend hoher Signalauflösung wird dem Breath-by-Breath-Verfahren der Vorzug gegeben.

Die Ausgangsdaten des Fluß- bzw. Volumensensors 2 und der CO₂/O₂-Sensoren 4, 5 gelangen auf eine Einheit zur Meßwertgewinnung 6, die mit einem Prozessor zur Steuerung des Meßablaufs sowie zur Meßsignalauswertung 8 in Verbindung steht. Die Steuereinheit 8 ist ebenfalls in der Lage, eine Gaspumpe 9 mit Signalen zu versorgen, so daß die gewünschten Strömungswerte in der Absaugstrecke 3 erhalten bleiben bzw. erreicht werden.

Zusätzlich besteht die Möglichkeit, der Meßwertgewinnungseinheit 6 Signale von weiteren Sensoren wie z.B. für die Umgebungstemperatur 10 und/oder die Gasfeuchte 11 zuzuführen. Letztgenante Sensorwerte können zur Kalibrierung bzw. Korrektur der von den Sensoren 4 und 5 erhaltenen Atemgasanalyse herangezogen werden.

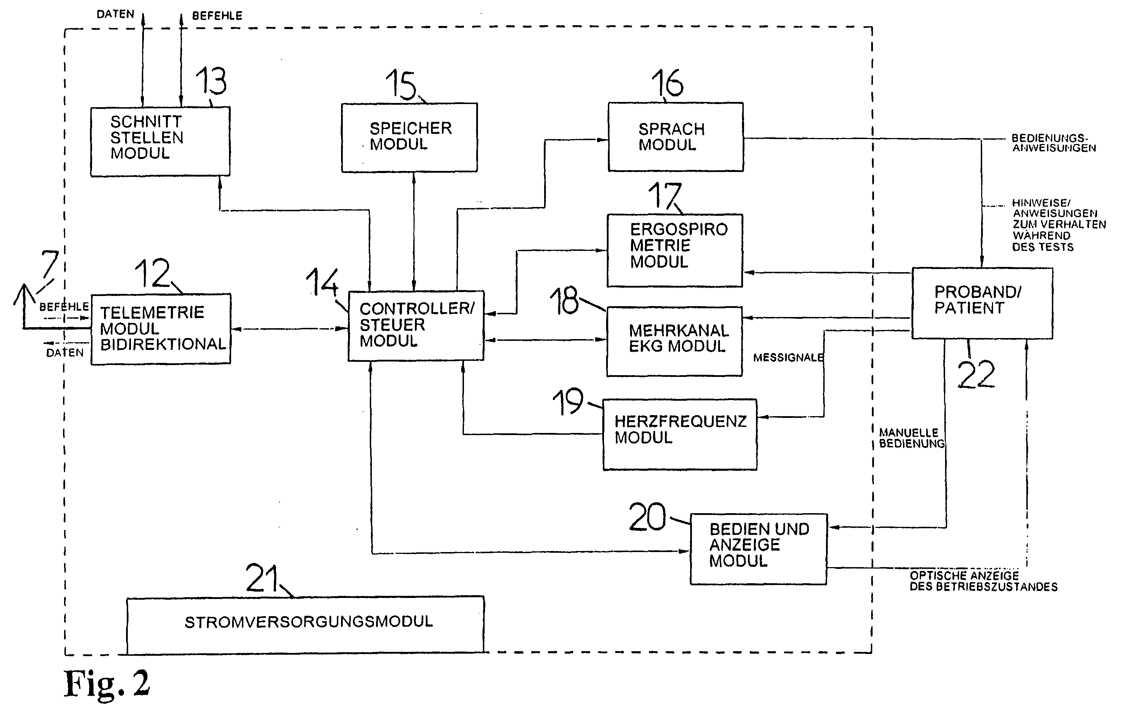

Gemäß Blockschaltbild nach Fig. 2 enthält die tragbare Meßeinheit ein Telemetriemodul 12 mit Sende- und Empfangsantenne 7, die in der Lage ist, eine bidirektionale Verbindung zur Telemetrieeinheit der Basisstation aufzubauen.
Ein Schnittstellenmodul 13 dient der unmittelbaren Kommunikation mit einem anschließbaren Personal-Computer bzw. einer computergestützten Basisstation oder einem anderen medizintechnischen Gerät.

Ein Signalverarbeitungsprozessor 14 dient der Abwicklung sämtlicher Kontroll- und Steueroperationen, aber auch zur Steuerung der Berechnung und Speicherung erhaltener Meßwerte in der tragbaren Meßeinheit. Hierfür ist am Signalverarbeitungsprozessor 14 ein Speichermodul 15 angeschlossen und es besteht eine weitere Verbindung zu einem Sprachmodul 16 zur Ausgabe von Bedienungsanweisungen bzw. zur akustischen Darstellung von Meßwerten. Die Ergospirometriekomponente 17 wird übergeordnet vom Signalprozessor 14 bedient und steht eingangsseitig gemäß Fig. 1 mit dem Probanden bzw. Patienten 22 in Verbindung. Weitere Meßsignale gelangen auf ein Mehrkanal-EKG 18 und es wird zusätzlich die momentane Herzfrequenz des Probanden über einen Herzfrequenzdetektor 19 ermittelt.

Bedien- und Anzeigemittel 20 sind in der Lage, über den Signalverarbeitungsprozessor Meßabläufe auszulösen bzw. momentane Zustände der Meßeinheit anzugeben, so daß der Proband in ausreichender Weise informiert ist und ggf. manuell in den Meßverlauf eingreifen kann.

Ein Stromversorgungsmodul 21 enthält sowohl Sekundärelemente als auch eine Schaltung zur elektronischen Spannungsstabilisierung mit dem Ziel des Minimierens des Gesamtstromverbrauchs der Meßeinheit, indem beispielsweise nicht benötigte Module oder Einheiten in einen sogenannten Sleep-Modus dann überführt werden können, wenn deren Funktion unter Berücksichtigung des aktuellen Meßverlaufs nicht benötigt werden.

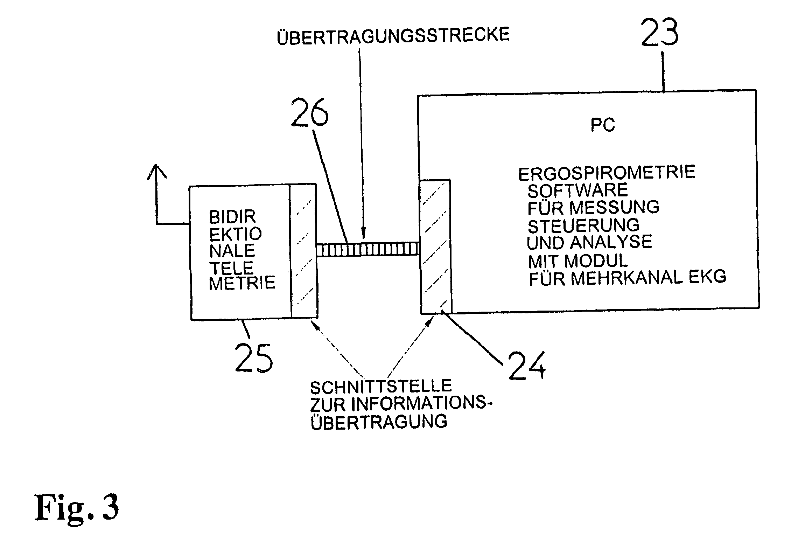

Die Basisstation nach Fig. 3 besteht aus einem Personal-Computer 23 mit einer Schnittstelle 24, über die eine Verbindung zu einer Telemetrieeinheit 25 herstellbar ist. Die Telemetrieeinheit 25 kann durch eine z.B. Infrarot- oder drahtgebundene Ausführung der Übertragungsstrecke 26 vom Personal-Computer 23 abgesetzt angeordnet werden, bevorzugt mit dem Zweck einer optimierten Position zum Aufbau der eigentlichen Hochfrequenz-Telemetrieverbindung.

Mittels Prozessor in der Meßeinheit oder über den Personal-Computer 23 und dort vorgesehene Software besteht die Möglichkeit, eine automatische Frequenzwahl für die Telemetrie zu aktivieren, um z.B. nach Scannen des Frequenzbands und/oder einer Testdatenprozedur diejenige Frequenz auszuwählen und festzulegen, bei der mit erwarteter optimaler Übertragungsqualität gearbeitet werden kann.
Beispielsweise ist hierbei ein Frequenzbereich von im wesentlichen 430 bis 470 MHz überstreichbar, wobei selbstverständlich auch andere Frequenzbereiche je nach länderspezifischer Freigabe vorgesehen sein können.

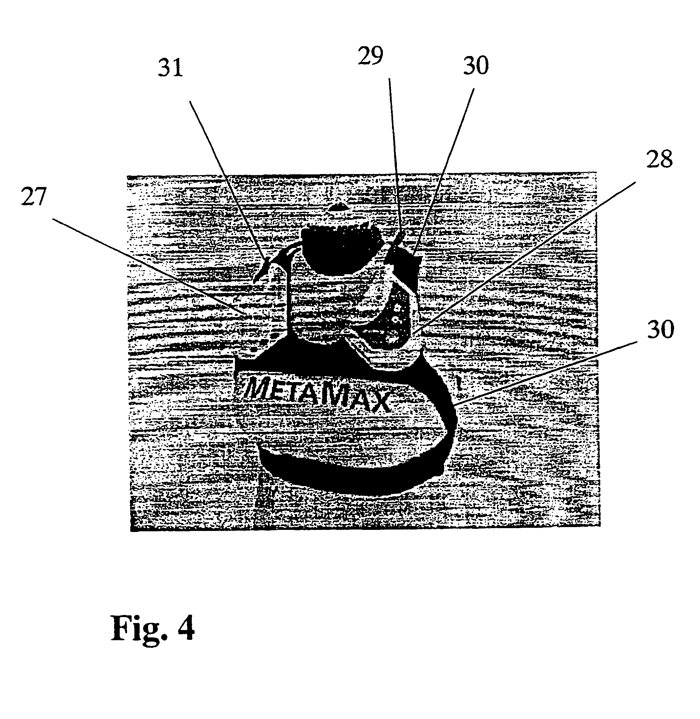

Bei einem Ausführungsbeispiel der tragbaren Meßeinheit nach Fig. 4 ist diese in Form zweier Gehäusekomponenten 27 und 28 ausgeführt, wobei die Gehäusekomponenten 27 und 28 über ein Kabel 29 miteinander elekrisch verbunden sind.

Ein spezieller Tragegurt 30 wird am Körper des Probanden befestigt und dient der Aufnahme der Gehäusekomponenten 27 und 28 in einem Bereich am Oberkörper, dort vorzugsweise in Schulter/Schlüsselbeinnähe.

Die Gehäusekomponente 27 enthält u.a. das Telemetriemodul mit Antenne 31. Im einzelnen nicht dargestellte Bedien- und Anzeigelemente sowie Steckverbinder dienen der Betätigung der Meßeinheit respektive dem Anschluß eines Ohrhörers zur Sprachausgabe. Dadurch, daß die Gehäusekomponenten 27 und 28 am Oberkörper in der bildlich dargestellten Weise befestigt sind, ist der Proband nur minimal beim Ausüben bestimmter Tätigkeiten, die bei Belastungstests erforderlich sind, behindert und durch die günstige Lage der Antenne 31 ergibt sich eine optimale Telemetriestrecke.

Alles in allem gelingt es mit der Erfindung, ein neuartiges, weitergebildetes mobiles Ergospirometriesystem anzugeben, mit dessen Hilfe unter weitgehend natürlichen Bedingungen und Belastungssituationen Atemgasanalysen in Verbindung mit der Ermittlung kardiologischer Meßwerte durchgeführt werden können, wobei die versuchsleitende Person in die Lage versetzt ist, über eine bidirektionle Telemetrie-Übertragungsstrecke nicht nur Meßdaten online zu erfassen und zu bewerten, sondern darüber hinaus auch unmittelbar durch Auslösen von Verhaltenshinweisen oder mittels Eingriff in die Steuerung der Meßeinheit den Versuchsverlauf beeinflussen kann.

### Bezugszeichenliste

- 1: Maske/Mundstück
- 2: Fluß/Volumensensor
- 3: Absaugstrecke
- 4; 5: CO₂/O₂-Sensor
- 6: Meßwerte-Gewinnungseinheit
- 7: Sende/Empfangsantenne des Telemetriemoduls
- 8: Steuereinheit
- 9: Pumpe
- 10: Temperatursensor
- 11: Feuchtesensor
- 12: Telemetriemodul
- 13: Schnittstellenmodul
- 14: Signalverarbeitungsprozessor
- 15.: Speichermodul
- 16: Sprachmodul
- 17: Ergospirometriekomponente bzw. -modul
- 18: Mehrkanal-EKG-Modul
- 19: Herzfrequenzdetektor
- 20: Bedien-/Anzeigeelemente
- 21: Stromversorgung
- 22: Proband
- 23: Personal-Computer
- 24: Schnittstelle
- 25: Telemetrieeinheit
- 26: Übertragungsstrecke
- 27; 28: Gehäusekomponente
- 29: Verbindungskabel
- 30: Tragegurt
- 31: Antenne

## Patentansprüche

1. Mobiles Ergospirometriesystem, umfassend eine am Probanden fixierbare, tragbare Meßeinheit mit Mundstück oder Maske (1) zur Atemgasabnahme, Gasvolumen- oder Mengensensor (2) sowie Sensoren (4,5) zur Bestimmung der CO₂/O₂-Konzentration im Atemgas, einen Signalverarbeitungsprozessor (14) und ein Telemetriemodul (12) sowie eine computergestützte Basisstation mit Telemetrieeinheit (25) zum Aufbau einer drahtlosen Verbindung mit dem Telemetriemodul (12),
**dadurch gekennzeichnet, daß**
über das Telemetriemodul (12) zur Telemetrieeinheit (25) eine bidirektionale Daten- und Befehlsübertragungsstrecke errichtet wird, wobei hierfür jeweils eine Sender- und Empfängerbaugruppe vorgesehen ist, so daß zusätzlich von der Basisstation aus online dem Probanden Informationen oder Aufforderungen zur Bedienung der Meßeinheit und/oder zur Gestaltung eines Versuchsverlaufs übermittelbar sind, wobei der Signalverarbeitungsprozessor (14) empfangene, codierte Befehle auf ein Sprachmodul (16) zur Umsetzung und Audioausgabe von Verhaltenshinweisen oder dergleichen Anweisungen leitet.

2. Mobiles Ergospirometriesystem nach Anspruch 1,
**dadurch gekennzeichnet, daß**
die Telemetrieeinheit (25) der Basisstation eine automatische Frequenzwahleinrichtung aufweist, um nach Scannen des Frequenzband und/oder einer Testdaten-Ubertragungsprozedur eine Telemetriefrequenz mit erwarteter optimaler Ubertragungsqualität festzulegen.

3. Mobiles Ergospirometriesystem nach Anspruch 1 oder 2,
**dadurch gekennzeichnet, daß**
die Meßeinheit zusätzlich einen Herzfrequenzdetektor (19) sowie ein mehrkanaliges EKG-Modul (18) aufweist, wobei die Erfassung und Speicherung der ergospirometrischen und der kardiologischen Meßwerte mittels des Signalprozessors (14) synchronisiert erfolgt.

4. Mobiles Ergospirometriesystem nach Anspruch 3,
**gekennzeichnet durch**
einen Speicher (15) zur Datensicherung in der Meßeinheit, wobei im Speicher (15) Datensätze im definierten Format aus atemzugsbezogenen Meßdaten für jeden Atemzug und/oder für sämtliche oder ausgewählte Meßgrößen die kompletten Meßdatenverläufe über wählbare Intervalle zu wählbaren Startzeiten abgelegt werden.

5. Mobiles Ergospirometriesystem nach Anspruch 4,
**dadurch gekennzeichnet, daß**
mittels des Signalprozessors (14) eine Berechnung der Ergospirometriegrößen aus den gewonnenen Daten erfolgt, wobei diese berechneten Daten im Speicher (15) abgelegt und/oder dem Probanden darstell- oder akustisch übermittelbar sind.

6. Mobiles Ergospirometriesystem nach einem der vorangegangenen Ansprüche,
**dadurch gekennzeichnet, daß**
der Signalprozessor (14) mit oder ohne externen Befehl aus vorliegenden Meßdaten und/oder dem Versuchsverlauf Verhaltenshinweise ermittelt und über das Sprachmodul (16) an den Probanden ausgibt.

7. Mobiles Ergospirometriesystem nach einem der vorangegangenen Ansprüche,
**dadurch gekennzeichnet, daß**
das Sprachmodul (16) einen digitalen Speicher mit dort abgelegten Worten, Wortgruppen oder sonstigen akustisch darstellbaren Informationen aufweist.

8. Mobiles Ergospirometriesystem nach Anspruch 7,
**dadurch gekennzeichnet, daß**
mittels des Signalprozessors (14) aus den im digitalen Speicher abgelegten Worten oder Wortgruppen jeweils aktuelle befehlsorientierte Wortverbindungen ausgewählt, verknüpft und gesteuert ausgegeben werden.

9. Mobiles Ergospirometriesystem nach einem der vorangegangenen Ansprüche,
**dadurch gekennzeichnet, daß**
die Meßeinheit Sensoren zur Bestimmung der Umgebungstemperatur (10) und/oder der Atemgasfeuchte (11) und/oder weiterer relevanter Größen, wie z.B. Differenzdruckwerte aufweist, wobei diese Sensorwerte zur Kalibrierung und Korrektur der Atemgassensorwerte herangezogen werden.

10. Mobiles Ergospirometriesystem nach einem der vorangegangenen Ansprüche,
**dadurch.gekennzeichnet, daß**
die tragbare Meßeinheit eine weitere Schnittstelle (13) zum Datenaustausch mit der Basisstation, einem Personal-Computer oder einem medizintechnischen Gerät aufweist.

11. Mobiles Ergospirometriesystem nach Anspruch 1,
**dadurch gekennzeichnet, daß**
die Basisstation eine Schnittstelle (24) zur Herstellung einer Verbindung mit einer als Zusatzbaugruppe ausgeführten, bidirektionalen Telemetrieeinheit (25) umfaßt.

12. Mobiles Ergospirometriesystem nach einem der vorangegangenen Ansprüche,
**dadurch gekennzeichnet, daß**
die tragbare Meßeinheit aus zwei Gehäusekomponenten (27,28) besteht, die mittels eines Kabels (29) verbunden sind, und daß über einen Tragegurt (30) oder ein Tragegestell die Gehäusekomponenten (27,28) am Oberkörper des Probanden jeweils in Schulter/Schlüsselbeinnähe plaziert sind.

## Claims

1. A mobile ergospirometry system, comprising a portable measuring unit which may be attached at the test person, with a mouthpiece or mask (1) for the intake of respiratory gas, a gas volume or quantity sensor (2) as well as sensors (4, 5) for the determination of the CO₂/O₂ concentration in the respiratory gas, a signal processing processor (14) and a telemetry module (12) a well as a computer-aided base station with a telemetry unit (25) for establishing a wireless connection with the telemetry module (12),
**characterised in that**
a bidirectional data and command transmission link is established via the telemetry module (12) to the telemetry unit (25), with a transmitter and a receiver assembly each being provided for this, so that additional information or prompts for the operation of the measuring unit and/or for the structuring of a test sequence may be transmitted online from the base station to the test person, with the signal processing processor (14) directing received encoded commands to a voice module (16) for conversion and audio output of behaviour comments or similar instructions.

2. The mobile ergospirometry system according to Claim 1,
**characterised in that** the telemetry unit (25) of the base station comprises an automatic frequency selection device in order to establish a telemetry frequency with expected optimum transmission quality after scanning the frequency band and/or a test data transmission procedure.

3. The mobile ergospirometry system according to Claim 1 or 2,
**characterised in that**
the measuring unit additionally comprises a heart rate detector (19) as well as a multichannel ECG module (18), with the detection and storage of the ergospirometric and cardiologic measuring values being effected in synchronism by means of the signal processor (14).

4. The mobile ergospirometry system according to Claim 3,
**characterised by**
a memory (15) for data saving in the measuring unit, with data records in the defined format being stored from breath-related measuring data for each breath and/or for all or selected measuring quantities the complete measuring data characteristics being stored via selectable intervals at selectable start times.

5. The mobile ergospirometry system according to Claim 4,
**characterised in that**
a computation of the ergospirometry quantities is made from the obtained data by means of the signal processor (14), with this data being stored in the memory (15) and/or being transmitted to the test person for display or acoustically.

6. The mobile ergospirometry system according to one of the previous claims,
**characterised in that**
the signal processor (14) with or without external command determines behaviour comments from the existing measuring data and/or the test sequence and outputs them to the test person via the voice module (16).

7. The mobile ergospirometry system according to one of the previous claims,
**characterised in that**
the voice module (16) comprises a digital memory with words, word groups, or other information which may be presented acoustically.

8. The mobile ergospirometry system according to Claim 7,
**characterised in that**
current command-oriented word combinations are selected, linked, and output in a controlled manner from the words or word groups stored in the memory by means of the signal processor (14).

9. The mobile ergospirometry system according to one of the previous claims,
**characterised in that**
the measuring unit comprises sensors for determining the ambient temperature (10) and/or the respiratory gas humidity (11) and/or further relevant quantities such as e. g. differential pressure values, with these sensor values being utilised for the calibration and correction of the respiratory gas sensor values.

10. The mobile ergospirometry system according to one of the previous claims,
**characterised in that**
the portable measuring unit comprises a further interface (13) for data exchange with the base station, a personal computer, or a medical technical instrument.

11. The mobile ergospirometry system according to Claim 1,
**characterised in that**
the base station comprises an interface (24) for establishing a connection with a bidirectional telemetry unit which is configured as an auxiliary assembly.

12. The mobile ergospirometry system according to one of the previous claims,
**characterised in that**
the portable measuring unit consists of two housing components (27, 28) which are connected by means of a cable (29), and the housing components (27, 28) are positioned on the upper part of the test person's body in the proximity of the shoulder/clavicula each by means of a carrier belt (30) or a carrier frame.

## Revendications

1. Système mobile d'ergospirométrie comportant une unité de mesure portable pouvant être fixée sur la personne de test et munie d'un bec ou d'un masque (1) pour le prélèvement de gaz respiratoire, d'un capteur de volume ou de quantité de gaz (2) ainsi que de capteurs (4, 5) pour déterminer la concentration de CO₂/O₂ dans le gaz respiratoire, d'un processeur de traitement des signaux (14) et d'un module de télémétrie (12) ainsi que d'une station de base assistée par ordinateur avec une unité de télémétrie (25) pour établir une liaison sans fil avec le module de télémétrie (12), **caractérisé en ce qu'**il est érigé vers l'unité de télémétrie (25), via le module de télémétrie (12), un trajet de transmission bidirectionnelle de données et d'instructions pour lequel à cet effet un groupe structurel émetteur/récepteur est prévu respectivement, de sorte qu'il est possible de transmettre en ligne, depuis la station de base à la personne de test, des informations ou des ordres pour manipuler l'unité de mesure et/ou pour conditionner un déroulement de test, le processeur de traitement des signaux (14) amenant les instructions codées reçues vers un module vocal (16) pour la transposition et la sortie audio des avis concernant le comportement ou des instructions analogues.

2. Système mobile d'ergospirométrie selon la revendication 1, **caractérisé en ce que** l'unité de télémétrie (25) de la station de base présente un dispositif automatique de sélection de fréquence afin de fixer une fréquence télémétrique avec une qualité de transmission optimale attendue, après le balayage de la bande de fréquence et/ou après une procédure de transmission de données de test.

3. Système mobile d'ergospirométrie selon l'une ou l'autre des revendications 1 et 2, **caractérisé en ce que** l'unité de mesure comprend en supplément un détecteur de fréquence cardiaque (19) ainsi qu'un module d'électrocardiogramme à plusieurs canaux (18), la saisie et la mémorisation des valeurs de mesure ergospirométriques et cardiologiques ayant lieu de manière synchronisée au moyen du processeur de signaux (14).

4. Système mobile d'ergospirométrie selon la revendication 3, **caractérisé par** une mémoire (15) pour la sauvegarde des données dans l'unité de mesure, des jeux de données étant enregistrés dans la mémoire (15) dans un format défini à partir des données de mesure qui se réfèrent à la respiration pour chaque cycle respiratoire et/ou pour toutes les grandeurs mesurées ou pour des grandeurs mesurées sélectionnées, ainsi que les courbes des données de mesures complètes sont mémorisées sur des intervalles à choisir à des instants de départ à choisir.

5. Système mobile d'ergospirométrie selon la revendication 4, **caractérisé en ce qu'**un calcul des grandeurs ergospirométriques a lieu au moyen du processeur de signaux (14) à partir des données récupérées, lesdites données calculées étant enregistrées dans la mémoire (15) et/ou pouvant être transmises à la personne de test par représentation ou de manière acoustique.

6. Système mobile d'ergospirométrie selon l'une des revendications précédentes, **caractérisé en ce que** le processeur de signaux (14) détermine des avis concernant le comportement avec ou sans instruction externe à partir des données de mesures existantes et/ou à partir du déroulement de test, et les exprime à l'attention de la personne de test via le module vocal (16).

7. Système mobile d'ergospirométrie selon l'une des revendications précédentes, **caractérisé en ce que** le module vocal (16) comprend une mémoire numérique dans laquelle sont enregistrés des mots, des groupes de mots ou d'autres informations pouvant être reproduites de manière acoustique.

8. Système mobile d'ergospirométrie selon la revendication 7, **caractérisé en ce qu'**au moyen du processeur de signaux (14), il est respectivement possible de sélectionner, de chaîner et d'exprimer de manière commandée des chaînages actuels de mots orientés aux instructions, à partir des mots ou des groupes de mots enregistrés dans la mémoire numérique.

9. Système mobile d'ergospirométrie selon l'une des revendications précédentes, **caractérisé en ce que** l'unité de mesure présente des capteurs pour déterminer la température ambiante (10) et/ou l'humidité du gaz respiratoire (11) et/ou d'autres grandeurs pertinentes, comme par exemple des valeurs de pression différentielle, ces valeurs de capteurs étant prises en considération pour le calibrage et la correction des valeurs de capteur du gaz respiratoire.

10. Système mobile d'ergospirométrie selon l'une des revendications précédentes, **caractérisé en ce que** l'unité de mesure portable comprend une autre interface (13) pour l'échange de données avec la station de base, avec un ordinateur personnel ou avec un appareil de technique médicale.

11. Système mobile d'ergospirométrie selon la revendication 1, **caractérisé en ce que** la station de base comporte une interface (24) pour établir une liaison avec une unité de télémétrie bidirectionnelle (25) réalisée comme groupe structurel supplémentaire.

12. Système mobile d'ergospirométrie selon l'une des revendications précédentes, **caractérisé en ce que** l'unité de mesure portable est constituée de deux composants de boîtier (27, 28) qui sont reliés au moyen d'un câble (29) et **en ce que** les composants de boîtier (27, 28) sont placés respectivement sur le torse de la personne de test à proximité de l'épaule/la clavicule au moyen d'une courroie de port (30) ou d'une armature de port.
